# EUROPEAN PATENT SPECIFICATION

(11) **EP 1 454 915 B1**
(45) Date of publication and mention of the grant of the patent: **12.03.2008**
(21) Application number: 03019583.8
(22) Date of filing: 03.09.2003
(51) Int. Cl.: C07K 14/705, C12N 15/09, A01K 67/02

(54) **Polynucleotides, polypeptides and method for screening for useful dog candidates**
Polynukleotide, Polypeptide und Methoden, um nützliche Hunde zu testen
Polynucleotides, polypeptides et methode pour tester des chiens candidats utiles

(30) Priority: 24.12.2002 JP 2002373006
(43) Date of publication of application: 08.09.2004
(73) Proprietor: President of Gifu University, Gifu-shi, Gifu 501-1193 (JP)
(72) Inventor: Murayama, Miho, 1-1, Yanagido Gifu-shi Gifu 501-1193 (JP); Ito, Shin'ichi, 1-1, Yanagido Gifu-shi Gifu 501-1193 (JP)
(74) Representative: Reinhard - Skuhra - Weise & Partner

(56) References cited:
- US-B1- 6 486 310
- NIIMI YOUKO ET AL: "Allelic variation of the D4 dopamine receptor polymorphic region in two dog breeds, Golden retriever and Shiba" JOURNAL OF VETERINARY MEDICAL SCIENCE, vol. 61, no. 12, December 1999 (1999-12), pages 1281-1286, XP009032484 ISSN: 0916-7250
- NIIMI Y ET AL: "Breed differences in allele frequency of the dopamine receptor D4 gene in dogs" JOURNAL OF HEREDITY, vol. 92, no. 5, September 2001 (2001-09), pages 433-436, XP009032482 ISSN: 0022-1503
- INOUE-MURAYAMA MIHO ET AL: "Sequence comparison of the dopamine receptor D4 exon III repetitive region in several species of the order Carnivora" JOURNAL OF VETERINARY MEDICAL SCIENCE, vol. 64, no. 8, August 2002 (2002-08), pages 747-749, XP009032481 ISSN: 0916-7250
- ITO HIDEYUKI ET AL: 'Allele frequency distribution of the canine dopamine receptor D4 gene exon III and I in 23 breeds' JOURNAL OF VETERINARY MEDICAL SCIENCE vol. 66, no. 7, July 2004, pages 815 - 820

## Description

The present invention relates to a method for screening for useful dog candidates such as candidates for seeing-eye dogs, disaster rescue dogs, assisting dogs, narcotics detection dogs, watchdogs, and pet dogs based on the relationship between genetic polymorphism of the dopamine receptor D4 gene in dogs and the behavioral traits of those dogs.

The correlation between genetic polymorphism of the dopamine receptor D4 gene in dogs and behavioral traits have been reported with respect to nine types of alleles in exon 3 region of said gene (Inoue-Miho Murayama, et al., "Association between the dopamine receptor D4 gene polymorphism and behavioral trait in dogs", DNA Polymorphism, Toyo Publishing, June 15, 2002, Vol. 10, pp. 64-70).

An object of the present invention is to provide polynucleotides and polypeptides that can be used to easily screen for useful dog candidates having genetic aptitudes beneficial to people, and a method for screening for useful dog candidates that is different from methods described in the literature.

Other aspects and advantages of the invention will become apparent from the following description, taken in conjunction with the accompanying drawings, illustrating by way of example the principles of the invention.

The following provides a detailed explanation of concrete embodiments of the present invention.

A polynucleotide of an embodiment of the present invention is composed of a nucleotide sequence represented by SEQ ID NO: 1, SEQ ID NO: 3, SEQ ID NO: 5, SEQ ID NO: 7, SEQ ID NO: 8, SEQ ID NO: 9, or a nucleotide sequence that is substantially identical to one of those sequences. Substantially identical refers to single nucleotide polymorphisms (SNP) or SNP observed in corresponding genes in different species of organisms. In addition, nucleotide sequences substantially identical to the nucleotide sequences represented by SEQ ID NO: 1, SEQ ID NO: 3, and SEQ ID NO: 5 may contain sequences that code for amino acid sequences represented by SEQ ID NO: 2, SEQ ID NO: 4, and SEQ ID NO: 6, respectively. In the following explanation, a polynucleotide composed of a nucleotide sequence represented by SEQ ID NO: n (where n = 1, 3, 5, 7, 8, or 9), or a sequence substantially identical to that sequence, will be described as polynucleotide n.

Polynucleotides 3 and 5 both comprise exon 1 of the dopamine receptor D4 (DRD4) gene of Beagle dogs. Namely, polynucleotide 3 and polynucleotide 5 are in a relationship of genetic polymorphism with respect to the above exon 1. Polynucleotide 1 is located from the 61st to 84th bases from the 5'-terminal of polynucleotide 3. Polynucleotide 5 consists of nucleotide sequence that is deprived of continuous 24 bases of polynucleotide·3 and is the short form S of polynucleotide 3. The continuous 24 bases comprise polynucleotide 1. The nucleotide sequences of polynucleotides 1, 3, and 5 are all sequences that are conserved among all breeds of dogs, and differences in sequences that go beyond the degree of SNP are not observed.

Polynucleotides 8 and 9 both comprise intron 2 of the DRD4 gene of Beagle dogs. Namely, polynucleotide 8 and polynucleotide 9 are in a relationship of genetic polymorphism with respect to the above intron 2. Polynucleotide 7 is located from the 50th to 66th bases from the 5'-terminal of polynucleotide 8. Polynucleotide 9 consists of nucleotide sequence that is deprived of continuous 17 bases of polynucleotide 8 and is the short form P of polynucleotide 8. The continuous 17 bases comprise polynucleotide 7. The nucleotide sequences of polynucleotides 7 through 9 are all sequences that are conserved among all breeds of dogs, and differences in sequences that go beyond the degree of SNP are not observed.

Polynucleotide 1, 3, 5, 7, 8, or 9 can be inserted in the recombinant vectors or the plasmids in the embodiment of the present invention. Transformants of the embodiment are obtained by introducing the recombinant vectors or plasmids into various host cells. Antisense DNAs or antisense RNAs of the embodiment have complementarity with respect to at least one portion of the polynucleotide 1, 3, 5, 7, 8, or 9.

These polynucleotides can be used as screening probes or identifying probes used in southern hybridization, northern hybridization, or in situ hybridization. At this time, these polynucleotides are normally labeled with radioisotopes or fluorescent pigments. These polynucleotides can also be used for DNA chips (microarrays).

A polypeptide of the embodiment of the present invention is composed of an amino acid sequence encoded by polynucleotide 1, 3, 5, 7, 8, or 9. Among these polypeptides, those composed of an amino acid sequence encoded by polynucleotide 1, 3, or 5, for example those composed of an amino acid sequence encoded by SEQ ID NO: 2, SEQ ID NO: 4, or SEQ ID NO: 6, are particularly useful. These polypeptides can be used for producing polyclonal antibodies, monoclonal antibodies, or hybridomas for the monoclonal antibodies. In particular, antibodies to polypeptide encoded by polynucleotide 1, 3, or 5 are useful for obtaining information relating to the expression of DRD4 protein encoded by DRD4 gene. These polypeptides can also be used for protein chips (microarrays).

The DRD4 protein is a G protein coupling receptor that bonds with neurotransmitters like dopamine and serotonin, and is involved in the expression of various psychogenesis by governing neural transmission. This DRD4 protein is a cell membrane protein having seven transmembrane sites. The N-terminal of the DRD4 protein is encoded by exon 1 (polynucleotide 3 or 5) located on the 5'-terminal side of the DRD4 gene. Intron 2 located downstream from the 3'-terminal of the exon 1 in contact with its 3'-terminal is comprised of polynucleotide 8 or 9.

A useful dog candidate of the embodiment of the present invention is an individual dog having genetic aptitudes that express behavioral traits beneficial to people. Examples of useful dog candidates include candidates for pets such as watchdogs and house dogs and candidates for service dogs such as seeing-eye dogs, disaster rescue dogs, assisting dogs, narcotics detection dogs, police dogs, dogs for the hearing impaired, animal herding dogs, and hunting dogs. These useful dog candidates are selected by matching their own genetic aptitudes with needs of an owner or user. The genetic aptitudes are determined based on the results of evaluating the aptitude evaluation parameters relating to various behavioral traits as shown in Tables 1 and 2 below. These genetic aptitudes are primarily determined genetically (congenitally).

**[Table 1]**

| No. | Aptitude Evaluation Parameters (Behavioral Traits) | Explanation |
|---|---|---|
| 1 | Aggression | Absence of aggressive or threatening behavior |
| 2 | Distraction (Dog) | Absence of excessive distraction with respect to other dogs |
| 3 | Distraction (Animal) | Absence of excessive distraction with respect to birds or cats |
| 4 | Distraction (Food) | Absence of excessive distraction with respect to food |
| 5 | Distraction (Scent) | Absence of excessive distraction with respect to scent |
| 6 | Excitability | Absence of excitability (hyperactivity) |
| 7 | Concentration | Able to perform tasks with a suitable degree of concentration |
| 8 | Suspicion | Absence of cautious suspicion (visual distraction) |
| 9 | Anxious | Absence of anxious behavior |
| 10 | Body sensibility | Favorable acceptance of physical contact |
| 11 | Sound sensibility | Absence of excessive reaction to sound |
| 12 | Noisiness | Absence of noisy behavior such as howling, barking and snorting |
| 13 | Willingness | Desire to perform a given task |
| 14 | Voice sensibility | Good response to voices |
| 15 | Obedience | Obedient to actions of people |

**[Table 2]**

| | Hart & Hart | | Tanabe and Yamazaki |
|---|---|---|---|
| No. | Aptitude Evaluation Parameters (Behavioral Traits) | No. | Aptitude Evaluation Parameters (Behavioral Traits) |
| 21 | Aggressiveness toward other dogs | 41 | Territory defensiveness |
| 22 | Defiance | 42 | Aggressiveness toward other dogs |
| 23 | Excessively playful | 43 | Defiance |
| 24 | Territory defensive | 44 | Controllability |
| 25 | Cautious howling | 45 | Cowardly |
| 26 | Biting of children | 46 | Ease of house training |
| 27 | Hypersensitivity | 47 | Obedience |
| 28 | Unnecessary howling | 48 | Introverted |
| 29 | Ordinary activity | 49 | Sociality |
| 30 | Friendliness towards people | 50 | Extroverted |
| 31 | Playfulness | 51 | Sociability |
| 32 | House training | 52 | Friendliness |
| 33 | Obedience | | |

The aptitude evaluation parameters shown in Table 1 are parameters emphasized when evaluating the behavioral traits of individual dogs prior to training by various types of dog trainers such as trainers of seeing-eye dogs. The evaluation parameters of Hart & Hart shown in Table 2 are listed in the literature describing an evaluation of behavioral traits to 10 levels based on a questionnaire given to veterinarians and dog trainers (Hart BL, Hart LA: The perfect puppy, W.H. Freeman and Company (USA) 1988). The evaluation parameters of Tanabe and Yamazaki also shown in Table 2 are listed in the literature describing a similar evaluation of behavioral traits to 5 levels (Tanabe, Y. and Yamazaki, K.: Differences in behavioral traits by dog breed based on a voluntary evaluation survey - Focusing on aptitude as a house dog, Journal of Veterinary Medicine JVM, Vol. 54, No. 1, January 2001 issue, pp. 9-14).

When screening useful dog candidates for watchdogs, dogs are selected that are evaluated as superior primarily for parameters Nos. 24, 25, and 41. When screening useful dog candidates for house dogs, dogs are selected that are evaluated as superior primarily for parameters Nos. 30 and 52. When screening for other pets, evaluation parameters matching owner preferences and compatibility are suitably taken into consideration. On the other hand, when screening useful dog candidates for service dogs, dogs are suitably selected that are superior with respect to those evaluation parameters suitable for the required roles. For example, in the case of welfare service dogs, such as seeing-eye dogs, assisting dogs, and dogs for the hearing impaired, dogs are primarily selected that are superior for at least one type of evaluation parameter from evaluation parameters Nos. 1 through 15, and preferably superior for all evaluation parameters Nos. 1 through 15. A summary of these relationships is shown in Table 3 below.

**[Table 3]**

| Type of useful dog candidate | Evaluation parameters (No.) for which candidate should be superior | Evaluation parameters (No.) for which candidates should not be superior |
|---|---|---|
| Watchdogs | 24, 25, 41 | 28, 30, 31 |
| House dogs | 30-33, 46, 47, 49, 51, 52 | 21-28, 41-45 |
| Seeing-eye dogs | 1-15 | 21-28, 41-45 |
| Assisting dogs | 1-15 | 21-28, 41-45 |
| Dogs for the | 1-15 | 21-28, 41-45 |
| hearing impaired | | |
| Disaster rescue | 1-15 | 21-28, 41-45 |
| dogs | | |
| Narcotics detection | 1,6-8,11,13,14 | 21-28, 41-45 |
| dogs | | |
| Police dogs | 6-8, 11, 13, 14 | 22,27 |
| Animal herding | 7, 13-15,29 | 22,27,43,45 |
| dogs | | |
| Hunting dogs | 29,33,47 | 22,27,28,31,43,45 |

In these useful dog candidates, there are three types of exon 1 of the DRD4 gene, which types are: homozygote S/S having polynucleotide 5 (short form S) in both alleles; heterozygote S/L having polynucleotide 5 in one allele and polynucleotide 3 (long form L) in another allele; and homozygote L/L having polynucleotide 3 in both alleles. Moreover, in the same useful dog candidates, there are three types of intron 2 of the DRD4 gene, which types are: homozygote P/P having polynucleotide 9 (short form P) in both alleles; heterozygote P/Q having polynucleotide 9 in one allele and polynucleotide 8 (long form Q) in another allele; and homozygote Q/Q having polynucleotide 8 in both alleles.

The genotypes of exon 1 and intron 2 (short form S and long form L, and short form P and long form Q) are each closely related to the behavioral traits of individual dogs. For example, dogs that are homozygotic S/S and heterozygotic S/L are superior with respect to evaluation parameters Nos. 2, 4, 5, 6, and 15 in Table 1 as compared with dogs that are homozygotic L/L having polynucleotide 3 in both alleles, and are suited for use as welfare service dogs such as seeing-eye dogs. In addition, when the short form S is compared with the long form L, dogs having the short form S easily express the behavioral traits of evaluation parameters Nos. 2, 3, 4, 5, 6, 7, 12, 15, 51, and 52, while dogs having the long form L easily express the behavioral traits of evaluation parameters Nos. 24, 41, 42, 43, and 44. Similarly, when the short form P is compared with the long form Q, dogs having the short form P easily express the behavioral trait of evaluation parameter No. 49, while dogs having the long form Q easily express the behavioral traits of evaluation parameters Nos. 21 and 22. In other words, since dogs having the short form S easily express behavioral traits particularly superior with respect to training performance, they are suited for use as service dogs such as welfare service dogs, disaster rescue dogs, narcotics detection dogs, and animal herding dogs, while conversely, dogs having the long form L are suited for use as watchdogs since they easily express behavioral traits associated with a high level of aggressiveness. Similarly, since dogs having the short form P easily express behavioral traits associated with superior sociality, they are suited for use as service dogs such as welfare service dogs, disaster rescue dogs, narcotics detection dogs, and animal herding dogs, while conversely, dogs having the long form Q are suited for use as watchdogs since they easily express behavioral traits associated with a high level of aggressiveness.

In the screening for useful dog candidates, the Polymerase Chain Reaction (PCR) method described below is used most easily. Namely, after extracting a genomic DNA containing the alleles of exon 1 or intron 2 of the DRD4 gene from a dog, a PCR reaction is carried out using a pair of PCR primers that bond to the vicinity of both terminals, and preferably to both terminals, of said alleles (exon 1 or intron 2) followed by confirmation of the lengths of the resulting PCR products by electrophoresis. It should be noted that, although arbitrary combinations able to be designed for amplifying the sequence (polynucleotide 1) from the 61st to 84th bases from the 5'-terminal of polynucleotide 3 can be employed for the pair of PCR primers that bond in the vicinity of both terminals of exon 1, a pair of PCR primers consisting of the nucleotide sequences represented by SEQ ID NO: 10 and SEQ ID NO: 11 are used preferably. Although arbitrary combinations able to be designed for amplifying the sequence (polynucleotide 7) from the 50th to 66th bases from the 5'-terminal of polynucleotide 8 can be employed for the pair of PCR primers that bond in the vicinity of both terminals of intron 2, a pair of PCR primers consisting of the nucleotide sequences represented by SEQ ID NO: 12 and SEQ ID NO: 13 are used preferably. In addition to the PCR method described above, southern hybridization method and northern hybridization method can be applied as the method for screening for useful dog candidates, and the alleles can also be investigated by directly determining the nucleotide sequences.

A kit for screening useful dog candidates of the embodiment of the present invention is for carrying out the screening for useful dog candidates described above extremely easily, and is provided with the pair of PCR primers that bond to the vicinity of both terminals, and preferably to both terminals, of exon 1 or intron 2.

A service dog of the embodiment of the present invention is an individual dog having superior genetic aptitudes for use as a seeing-eye dog, disaster rescue dog, assisting dog, narcotics detection dog, police dog, dog for the hearing impaired, animal herding dog, or hunting dog. These service dogs preferably have the short form S or short form P, since they easily express superior behavioral traits with respect to training performance. Namely, these service dogs are either dogs in which at least one of the alleles of exon 1 of the DRD4 gene is composed of polynucleotide 5, or dogs in which at least one of the alleles of intron 2 of the DRD4 gene is composed of polynucleotide 9. In addition, dogs having polynucleotide 5 for exon 1 of the DRD4 gene as well as polynucleotide 9 for intron 2 of the DRD4 gene are the most preferable.

According to the results of research on exon 3 of the DRD4 gene conducted by Inoue et al. as previously described, dog breeds belonging to groups A, B, and C having less aggressiveness and superior genetic aptitudes with respect to training performance are suitably used as service dogs. Namely, the dog breeds having less allele 498 for exon 3 are used particularly preferably. Examples of breeds belonging to group A include Boxer, Yorkshire terrier, German shepherd, Beagle, Golden retriever, Basset hound, and Pomeranian. Among these, Boxer, German shepherd, Beagle, or Golden retriever is used particularly preferably.

Examples of breeds belonging to group B include Bulldog, Shetland sheepdog, Miniature schnauzer, Cocker spaniel, Old English sheepdog, Labrador retriever, Chihuahua, Toy poodle, Maltese, Collie, and Pug. Among these, Bulldog, Shetland sheepdog, Miniature schnauzer, Cocker spaniel, Old English sheepdog, Labrador retriever, Toy poodle, Collie, or Pug is used particularly preferably. Examples of breeds belonging to group C include Standard poodle, Dalmatian, Welsh collie Pembrook, and Doberman.

Among these dog breeds, the dogs of group B are used preferably as service dogs since they are traditionally evaluated as being highly useful as service dogs, with Labrador retriever, Shetland sheepdog, Old English sheepdog, or Collie being used particularly preferably, and Labrador retriever being used the most preferably. In addition, German shepherds may also be used as police dogs since they are traditionally widely recognized as being useful as police dogs. In addition, since it is easy to provide a desired service dog inexpensively, a crossbreed, preferably a crossbreed between any of the above breeds, and particularly preferably a first-generation crossbreed (F1) of any of the above breeds, may also be used.

A method for screening for service dogs of the embodiment of the present invention investigates the alleles of exon 1 of the DRD4 gene, and screens for those dogs in which at least one of the alleles is comprised of polynucleotide 5. Since the selected dogs having a genotype of either homozygotic S/S or heterozygotic S/L for polynucleotide 5 have genetic aptitudes beneficial to people described above, they are suitable for being trained to be seeing-eye dogs or other service dogs. In addition, selected dogs having a genotype of homozygotic S/S for polynucleotide 5 are also used as parent animals for breeding service dogs, and male dogs in particular have a high value as breeding dogs.

Alternatively, as another method for screening for service dogs, the alleles of intron 2 of the DRD4 gene may be investigated, and those dogs in which at least one of the alleles is composed of polynucleotide 9 may be selected. In particular, selected dogs having a genotype of homozygotic P/P for polynucleotide 9 are used as parent dogs for breeding service dogs, and male dogs in particular have a high value as breeding dogs. In addition, the most preferable method of screening service dogs consists of investigating two types of alleles of exon 1 and intron 2 of the DRD4 gene, and selecting those dogs in which at least one of the alleles of exon 1 is composed of polynucleotide 5, and at least one of the alleles of intron 2 is composed of polynucleotide 9. Screening of these service dogs is most easily carried out by investigating the genotype of the alleles by carrying out the same procedure as that of the screening method for useful dog candidates previously described (PCR and electrophoresis staining) and then screening for candidate dogs useful as service dogs based on that genotype.

A breeding method of service dogs of the embodiment of the present invention consists of breeding dogs in which at least one of the alleles of exon 1 of the DRD4 gene is composed of polynucleotide 5. Since the bred dogs having a genotype of either homozygotic S/S or heterozygotic S/L for polynucleotide 5 have genetic aptitudes beneficial to people, they are suitable for being trained to be service dogs such as seeing-eye dogs. In addition, bred dogs having a genotype of homozygotic S/S for polynucleotide 5 can be used for additional breeding of service dogs. The method used to breed these service dogs consists of using for at least one of the parent dogs an adult dog that is homozygotic S/S for polynucleotide 5 previously screened by the screening method described above, or previously bred according to the breeding method described above.

Alternatively, as another method for breeding service dogs, a dog may be bred in which at least one of the alleles of intron 2 of the DRD4 gene is composed of polynucleotide 9. In particular, bred dogs having a genotype of homozygotic P/P for polynucleotide 9 can be used for additional breeding of service dogs. The method used to breed these service dogs consists of using for at least one of the parent dogs an adult dog that is homozygotic P/P for polynucleotide 9 previously screened by the screening method described above, or previously bred according to the breeding method described above. In addition, the most preferable method of raising the service dogs consists of breeding a dog in which at least one of the alleles of exon 1 of the DRD4 gene is composed of polynucleotide 5, and at least one of the alleles of intron 2 is composed of polynucleotide 9.

The following provides a description of the effects demonstrated by the above embodiments.

Since the screening method for useful dog candidates of the embodiment of the present invention is a screening method based on scientific grounds regarding the correlation between the genotypes of exon 1 or intron 2 and the behavioral traits (phenotypes) of dogs, dogs that match the needs of owners or users can be provided to them as a result of extremely accurate screening. Moreover, in these screening methods, since the compatibility of the useful dog candidates can be identified before they are provided to their owners or users, mismatches between owners or users and their dogs can be easily reduced before the dogs are provided to their owners or users. In addition, the use of PCR for the screening method results in greater convenience since useful dog candidates can be screened remarkably easily and in a short period of time.

Service dogs in which at least one allele of exon 1 of the DRD4 gene is composed of polynucleotide 5 easily express behavioral traits that are suitable for use as service dogs, such as being free of excessive distraction with respect to other dogs, food, and scent, being less likely to become overly excited, being able to perform a task with a suitable level of concentration, and being obedient to the actions of people. In addition, service dogs in which at least one of the alleles of intron 2 of the DRD4 gene is composed of polynucleotide 9 easily express behavioral traits suitable for service dogs associated with low levels of aggressiveness and defiance but ample sociality. These service dogs have a high latent potential for becoming superior service dogs since they are able to be trained while concentrating on the instructions given by trainers when undergoing training to become a seeing-eye dog or other service dog.

Moreover, since these service dogs enable the amount of time and labor spent on training to be easily reduced, it is easy to shorten training time. In addition, since these service dogs are rarely determined to be unacceptable after the start of training to become service dogs, the supply volume of service dogs can be easily increased. This screening method is used particularly effectively for the screening of seeing-eye dogs, disaster rescue dogs, assisting dogs, narcotics detection dogs, or police dogs that require a long training period and are in small supply relative to demand. In addition, the use of PCR during screening for service dogs results in greater convenience since service dogs can be screened remarkably easily and in a short period of time.

Dogs that are homozygotic S/S, in which both of the alleles of exon 1 of the DRD4 gene are composed of polynucleotide 5, can be used as at least one of the parent dogs during breeding of service dogs. In addition, dogs that are homozygotic P/P, in which both of the alleles of intron 2 of the DRD4 gene are composed of polynucleotide 9, can be used as at least one of the parent dogs during breeding of service dogs. The dogs being homozygotic S/S or homozygotic P/P are useful because they allow the easy and reliable breeding of superior service dogs that are homozygotic S/S, heterozygotic S/L, homozygotic P/P, or heterozygotic P/Q. In addition, since the parent dogs, and particularly male dogs, can be used repeatedly over and over for breeding, the supply volume of service dogs can be easily increased.

### [Examples]

The following provides an explanation of Examples implemented with the above embodiments and Comparative

### examples.

### <Survey of Genetic Polymorphism of Exon 1>

After sampling epidermal cells from the oral cavities of a plurality of Beagle dogs, the genomic DNA was extracted from those cells by a conventional column method. A PCR reaction was carried out on the resulting DNA using a pair of PCR primers that bond to both terminals of exon 1 of a human DRD4 gene. The pair of PCR primers is composed of the nucleotide sequences represented by SEQ ID NO: 10 and SEQ ID NO: 11. When electrophoresis was carried out with a fluorescent sequencer using the resulting PCR products, two lengths of PCR products, namely a long form L and a short form S, were present. When the respective nucleotide sequences of these two types of PCR products were investigated, the long form L was confirmed to be the nucleotide sequence represented by SEQ ID NO: 3, while the short form S was confirmed to be the nucleotide sequence represented by SEQ ID NO: 5.

### <Survey of Genetic Polymorphism of Intron 2>

After sampling epidermal cells from the oral cavities of a plurality of Beagle dogs, the genomic DNA was extracted from those cells. A PCR reaction was carried out on the resulting DNA using a pair of PCR primers for amplifying the sequence of intron 2 of a human DRD4 gene. The pair of PCR primers is composed of the nucleotide sequences represented by SEQ ID NO: 12 and SEQ ID NO: 13. When electrophoresis was carried out with a fluorescent sequencer using the resulting PCR products, two lengths of PCR products, namely a long form Q and a short form P, were present. When the respective nucleotide sequences of these two types of PCR products were investigated, they were confirmed to be the nucleotide sequences represented by SEQ ID NO: 8 and SEQ ID NO: 9, respectively.

### <Verification of Association Between Genetic Polymorphism of Exon 1 and Behavioral Traits - Part 1>

After sampling epidermal cells from the oral cavities of 70 Labrador retrievers raised at the Kansai Guide Dogs for the Blind Association (Japan), a genomic DNA was extracted from the cells. A PCR reaction was carried out on the resulting genomic DNA from each dog using a pair of PCR primers composed of the nucleotide sequences represented by SEQ ID NOs: 10 and 11. After performing electrophoresis using the resulting PCR products, the genotype of each dog was evaluated on the basis of the lengths of the PCR products and classified to one of three types consisting of homozygotic S/S (29 dogs), heterozygotic S/L (29 dogs), and homozygotic L/L (12 dogs).

On the other hand, each dog was scored for each of the evaluation parameters (Nos. 1 through 15) for behavioral traits shown in the previously described Table 1 by evaluating to one of five levels by two seeing-eye dog trainers. Evaluations using the five levels were conducted in accordance with evaluation standards consisting of extremely good (5), somewhat good (4), average (3), somewhat problematic (2), and extremely problematic (1) from the viewpoint of aptitude as a seeing-eye dog, namely whether or not the behavioral trait is beneficial to people.

Next, the dogs were divided into three groups consisting of homozygotic S/S, heterozygotic S/L, and homozygotic L/L, and analyzed for each of the evaluation parameters Nos. 1 through 15 by performing an analysis of variance using the number of dogs belonging to each genotype and the scores of those dogs. The resulting P values are shown in the Genotype column of Table 4. On the other hand, the same dogs were then grouped into two groups consisting of one group comprised of dogs having the short form S (S/S and S/L) and another group comprised of dogs not having the short form S (L/L), and analyzed by performing a similar analysis of variance. The resulting P values are shown in the Presence or Absence of S column of Table 4. In addition, the dogs were also grouped into two groups consisting of one group comprised of dogs not having the long form L (S/S) and another group comprised of dogs having the long form L (S/L and L/L), and analyzed by performing a similar analysis of variance. The resulting P values are shown in the Presence or Absence of L column of Table 4.

**[Table 4]**

| No. | Evaluation Parameters | Genotype (P value) | Presence or Absence of S (P value) | Presence or Absence of L (P value) |
|---|---|---|---|---|
| 1 | Aggression | 0.190 | 0.070 | 0.362 |
| 2 | Distraction (Dog) | 0.007(**) | 0.100 | 0.002(**) |
| 3 | Distraction (Animal) | 0.059 | 0.024(*) | 0.121 |
| 4 | Distraction (Food) | 0.006(**) | 0.146 | 0.001(**) |
| 5 | Distraction (Scent) | 0.000(***) | 0.028(*) | 0.000(***) |
| 6 | Excitability | 0.010(*) | 0.034(*) | 0.005(**) |
| 7 | Concentration | 0.019(*) | 0.314 | 0.004(**) |
| 8 | Suspicion | 0.818 | 0.525 | 0.791 |
| 9 | Anxious | 0.629 | 0.607 | 0.578 |
| 10 | Body sensibility | 0.563 | 0.283 | 0.633 |
| 11 | Sound sensibility | 0.943 | 0.762 | 0.975 |
| 12 | Noisiness | 0.068 | 0.020(*) | 0.428 |
| 13 | Willingness | 0.566 | 0.322 | 0.452 |
| 14 | Voice sensibility | 0.925 | 0.703 | 0.806 |
| 15 | Obedience | 0.015(*) | 0.025(*) | 0.012(*) |

Furthermore, those evaluation parameters for which a significant difference was detected in Table 4 and in the following analysis results of analysis of variance are indicated with a single asterisk (*) in the case of a level of significance of less than 5%, with a double asterisk (**) in the case of a level of significance of less than 1%, or with a triple asterisk (***) in the case of a level of significance of less than 0.1%. As a result, significant differences were detected in grouping by genotype and presence or absence of L for the six types of evaluation parameters of Nos. 2, 4, 5, 6, 7, and 15. In addition, significant differences were detected in grouping according to presence or absence of S for the five types of evaluation parameters of Nos. 3, 5, 6, 12, and 15. Moreover, the results of comparing whether or not the scores of any of the S/S, S/L, and L/L genotypes are higher for the six types of evaluation parameters for which a significant difference was detected in group according to genotype as mentioned above are shown in the Genotype column of Table 5. In addition, an overall assessment of the usefulness of the short form S and long form L as judged from those results is shown in the Allele column of the same table.

**[Table 5]**

| No. | Evaluation Parameter | Genotype | Allele |
|---|---|---|---|
| 2 | Distraction (Dog) | S/S > S/L > L>L | S > L |
| 4 | Distraction (Food) | S/S > S/L > L>L | S > L |
| 5 | Distraction (Scent) | S/S > S/L > L>L | S > L |
| 6 | Excitability | S/S > S/L > L>L | S > L |
| 7 | Concentration | S/S > S/L ≈ L>L | S > L |
| 15 | Obedience | S/S > S/L > L>L | S > L |

According to the results shown in Table 5, dogs having the short form S were indicated as having genetic aptitudes superior for use as seeing-eye dogs. Moreover, homozygotic S/S dogs were confirmed to be superior to heterozygotic S/L dogs. With respect to the six types of evaluation parameters shown in Table 5 in particular, it was confirmed that the scores of all homozygotic S/S dogs tended to be higher. Furthermore, although a data is not shown, the short form S was determined to be more useful than the long form L with respect to evaluation parameter No. 3 as well. On the other hand, in a preliminary experiment to investigate the relationship between feasibility of training as a seeing-eye dog and each of the above evaluation parameters, those dogs that passed training to be a seeing-eye dog had significantly higher scores for evaluation parameters Nos. 6 and 7 than those dogs that failed the same training. Incidentally, although these experiments were conducted using seeing-eye dogs, the experimental results obtained can be similarly applied to dogs including other service dogs and pets as well as seeing-eye dogs.

### <Verification of Association Between Genetic Polymorphism of Exon 1 and Behavioral Traits - Part 2>

Blood cells or epidermal cells in the oral cavities were sampled from roughly 30 breeds of several hundred dogs (four or more dogs of any one breed) that had been already evaluated in the literatures of Hart & Hart and Tanabe & Yamazaki for behavioral traits based on questionnaires given to veterinarians and dog trainers. A genomic DNA was extracted from the cells, and a PCR reaction was carried out using a pair of PCR primers composed of the nucleotide sequences represented by SEQ ID NOs: 10 and 11. After performing electrophoresis using the resulting PCR products, the genotype of each dog was determined on the basis of the lengths of the PCR products. Next, after grouping the determined genotypes into one group comprised of dogs having the short form S and another group comprised of dogs having the long form L for each breed, the dogs were analyzed by performing an analysis of variance using the number of dogs belonging to each group and the score of the breed to which the dogs belong. Those results are shown in Table 6.

### <Verification of Association Between Genetic Polymorphism of Intron 2 and Behavioral Traits>

Blood cells or epidermal cells in the oral cavities were sampled from roughly 25 breeds of several hundred dogs (four or more dogs of any one breed) that had been already evaluated for behavioral traits based on the questionnaires described above. A genomic DNA was extracted from the cells, and a PCR reaction was carried out using a pair of PCR primers composed of the nucleotide sequences represented by SEQ ID NOs: 12 and 13. After performing electrophoresis using the resulting PCR products, the genotype of each dog was determined on the basis of the lengths of the PCR products. Next, after grouping the determined genotypes into one group comprised of dogs having the short form P and another group comprised of dogs having the long form Q for each breed, the dogs were analyzed by performing an analysis of variance using the number of dogs belonging to each group and the score of the breed to which the dogs belong. Those results are shown in Table 6.

**[Table 6]**

| | Hart & Hart | | | Tanabe & Yamazaki | |
|---|---|---|---|---|---|
| No. | Exon 1 | Intron 2 | No. | Exon 1 | Intron 2 |
| | (32 breeds) | (26 breeds) | | (29 breeds) | (24 breeds) |
| 21 | | P < Q(*) | 41 | S < L(*) | |
| 22 | | P < Q(*) | 42 | S < L(*) | |
| 23 | | | 43 | S < L(*) | |
| 24 | S < L(*) | | 44 | S < L(**) | |
| 25 | | | 45 | | |
| 26 | | | 46 | | |
| 27 | | | 47 | | |
| 28 | | | 48 | | |
| 29 | | | 49 | | P> Q(*) |
| 30 | | | 50 | | |
| 31 | | | 51 | S > L(*) | |
| 32 | | | 52 | S > L(*) | |
| 33 | | | | | |

According to the results shown in Table 6, a trend was observed in which dogs having the short form S easily express mild-mannered behavioral traits, while dogs having the long form L easily express aggressive behavioral traits. Similarly, a trend was also observed in which dogs having the short form P easily express mild-mannered behavioral traits, while dogs having the long form Q easily express aggressive behavioral traits.

Therefore, the present examples and embodiments are to be considered as illustrative and not restrictive and the invention is not to be limited to the details given herein, but may be modified within the scope and equivalence of the appended claims.

## Claims

1. (Amended) A method for screening for useful dog candidates, **characterized by**:
a step of investigating the genotype of the allele in exon 1 of a dog dopamine receptor D4 gene, and
a step of selecting a dog with genetic aptitudes beneficial to people by making the selection based on that genotype, wherein the dogs with genetic aptitudes beneficial to people are service dogs, and the genotype is a homozygote or heterozygote of a polynucleotide composed of a nucleotide sequence represented by either SEQ ID NO: 5 or a sequence substantially identical to SEQ ID NO: 5.

2. (Amended) The method for screening for useful dog candidates according to claim 1, **characterized in that** the service dogs are seeing-eye dogs, disaster rescue dogs, assisting dogs, narcotics detection dogs, police dogs, dogs for the hearing impaired, animal herding dogs, or hunting dogs.

3. (Amended) A method for screening for useful dog candidates, **characterized by**:
a step of investigating the genotype of the allele in exon 1 of a dog dopamine receptor D4 gene, and
a step of selecting a dog with genetic aptitudes beneficial to people by making the selection based on that genotype, wherein the dogs with genetic aptitudes beneficial to people are watch dogs, and the genotype is a homozygote or heterozygote of a polynucleotide composed of a nucleotide sequence represented by either SEQ ID NO: 3 or a sequence substantially identical to SEQ ID NO: 3.

4. (Amended) The method for screening for useful dog candidates according to any one of claims 1 to 3, **characterized in that** the step of investigating the genotype comprises a step of amplifying the allele using a pair of PCR primers that bonds in the vicinity of both terminals of the allele, and a step of determining the genotype of the allele based on the lengths of the PCR products obtained by that step.

5. (Amended) The method for screening for useful dog candidates according to claim 4, **characterized in that** the pair of PCR primers is composed of the nucleotide sequences represented by SEQ ID NO: 10 and SEQ ID NO: 11.

6. (New) A kit for screening for useful dog candidates, **characterized by** a pair of PCR primers that bonds to the vicinity of both terminals of the allele in exon 1 of a dog dopamine receptor D4 gene.

7. (New) The kit according to claim 6, **characterized in that** the pair of PCR primers is composed of the nucleotide sequence represented by SEQ ID NO: 10 and SEQ ID NO: 11.

8. (Amended) A method for screening for useful dog candidates, **characterized by:**
a step of investigating the genotype of the allele in intron 2 of a dog dopamine receptor D4 gene, and
a step of selecting a dog with genetic aptitudes beneficial to people by making the selection based on that genotype.

9. (Amended) The method for screening for useful dog candidates according to claim 8, **characterized in that** the step of investigating the genotype comprises a step of amplifying the allele using a pair of PCR primers that bonds in the vicinity of both terminals of the allele, and a step of determining the genotype of the allele based on the lengths of the PCR products obtained by that step.

10. (Amended) The method for screening for useful dog candidates according to claim 9, **characterized in that** the pair of PCR primers is composed of the nucleotide sequences represented by SEQ ID NO: 12 and SEQ ID NO: 13.

11. (Amended) The method for screening for useful dog candidates according to any one of claims 8 to 10, **characterized in that** the dogs with genetic aptitudes beneficial to people are service dogs, and the genotype is a homozygote or heterozygote of a polynucleotide composed of a nucleotide sequence represented by either SEQ ID NO: 9 or a sequence substantially identical to SEQ ID NO: 9.

12. (Amended) The method for screening for useful dog candidates according to any one of claims 8 to 10, **characterized in that** the dogs having genetic aptitudes beneficial to people are watchdogs or house dogs.

13. (Amended) A kit for screening for useful dog candidates, **characterized by** a pair of PCR primers that bonds to the vicinity of both terminals of the allele in intron 2 of a dog dopamine receptor D4 gene.

14. (New) The kit according to claim 13, **characterized in that** the pair of PCR primers is composed of the nucleotide sequence represented by SEQ ID NO: 12 and SEQ ID NO: 13.

## Patentansprüche

1. Verfahren zum Screenen nach nützlichen Hundekandidaten, **gekennzeichnet durch**:
einen Schritt des Untersuchens des Genotyps des Allels in Exon 1 eines Hunde-Dopaminrezeptor-D4-Gens und
einen Schritt des Auswählens eines Hundes mit genetischer Eignung, die für den Menschen von Nutzen ist, wobei die Selektion auf diesem Genotyp basiert und wobei die Hunde mit genetischer Eignung, die für den Menschen von Nutzen ist, Servicehunde sind und der Genotyp ein Homozygot oder Heterozygot eines Polynukleotids ist, das aus einer Nukleotidsequenz besteht, die entweder **durch** die SEQ ID NO: 5 oder eine Sequenz, die im Wesentlichen mit der SEQ ID NO: 5 identisch ist, wiedergegeben ist.

2. Verfahren zum Screenen nach nützlichen Hundekandidaten nach Anspruch 1,
**dadurch gekennzeichnet, dass** die Servicehunde Blindenhunde, Katastrophenrettungshunde, Assistenzhunde, Drogenhunde, Polizeihunde, Hunde für Hörgeschädigte, Tierhütehunde oder Jagdhunde sind.

3. Verfahren zum Screenen nach nützlichen Hundekandidaten, **gekennzeichnet durch**:
einen Schritt des Untersuchens des Genotyps des Allels in Exon 1 eines Hunde-Dopaminrezeptor-D4-Gens und
einen Schritt des Auswählens eines Hundes mit der genetischen Eignung, die für den Menschen von Nutzen ist, wobei die Selektion auf diesem Genotyp basiert und wobei die Hunde mit genetischer Eignung, die für den Menschen von Nutzen ist, Wachhunde sind und der Genotyp ein Homozygot oder Heterozygot eines Polynukleotids ist, das aus einer Nukleotidsequenz besteht, die entweder **durch** die SEQ ID NO: 3 oder eine Sequenz, die im Wesentlichen mit der SEQ ID NO: 3 identisch ist, wiedergegeben ist.

4. Verfahren zum Screenen nach nützlichen Hundekandidaten nach einem der Ansprüche 1 bis 3, **dadurch gekennzeichnet, dass** der Schritt des Untersuchens des Genotyps einen Schritt des Amplifizierens des Allels unter Verwenden eines PCR-Primerpaares, das in der Nachbarschaft beider Termini des Allels bindet, und einen Schritt des Bestimmens des Genotyps des Allels auf Basis der Längen der mit diesem Schritt . erhaltenen PCR-Produkte umfasst.

5. Verfahren zum Screenen nach nützlichen Hundekandidaten nach Anspruch 4, **dadurch gekennzeichnet, dass** das PCR-Primerpaar aus den Nukleotidsequenzen besteht, die durch die SEQ ID NO: 10 und die SEQ ID NO: 11 wiedergegeben sind.

6. Kit zum Screenen nach nützlichen Hundekandidaten, **gekennzeichnet durch** ein PCR-Primerpaar, das in der Nachbarschaft der beiden Termini des Allels in Exon 1 eines Hunde-Dopaminrezeptor-D4-Gens bindet.

7. Kit nach Anspruch 6, **dadurch gekennzeichnet, dass** das PCR-Primerpaar aus den Nukleotidsequenzen besteht, die durch die SEQ ID NO: 10 und die SEQ ID NO: 11 wiedergegeben sind.

8. Verfahren zum Screenen nach nützlichen Hundekandidaten, **gekennzeichnet durch**:
einen Schritt des Untersuchens des Genotyps des Allels in Intron 2 eines Hunde-Dopaminrezeptor-D4-Gens und
einen Schritt des Auswählens eines Hundes mit genetischer Eignung, die für den Menschen von Nutzen ist, wobei die Auswahl auf diesem Genotyp basiert.

9. Verfahren zum Screenen nach nützlichen Hundekandidaten nach Anspruch 8, **dadurch gekennzeichnet, dass** der Schritt des Untersuchens des Genotyps einen Schritt des Amplifizierens des Allels unter Verwenden eines PCR-Primerpaares, das in der Nachbarschaft der beiden Termini des Allels bindet, und einen Schritt des Bestimmens des Genotyps des Allels auf Basis der Längen der mit diesem Schritt erhaltenen PCR-Produkte umfasst.

10. Verfahren zum Screenen nach nützlichen Hundekandidaten nach Anspruch 9, **dadurch gekennzeichnet, dass** das PCR-Primerpaar aus den Nukleotidsequenzen besteht, die durch die SEQ ID NO: 12 und die SEQ ID NO: 13 wiedergegeben sind.

11. Verfahren zum Screenen nach nützlichen Hundekandidaten nach einem der Ansprüche 8 bis 10, **dadurch gekennzeichnet, dass** die Hunde mit genetischer Eignung, die für den Menschen von Nutzen ist, Servicehunde sind und dass der Genotyp ein Homozygot oder Heterozygot eines Polynukleotids ist, das aus einer Nukleotidsequenz besteht, die entweder durch die SEQ ID NO: 9 oder eine Sequenz, die im Wesentlichen mit der SEQ ID NO: 9 identisch ist, wiedergegeben ist.

12. Verfahren zum Screenen nach nützlichen Hundekandidaten nach einem der Ansprüche 8 bis 10, **dadurch gekennzeichnet, dass** die Hunde mit genetischer Eignung, die für den Menschen von Nutzen ist, Wachhunde oder Haushunde sind.

13. Kit zum Screenen nach nützlichen Hundekandidaten, **gekennzeichnet durch** ein PCR-Primerpaar, das in der Nachbarschaft der beiden Termini des Allels in Intron 2 eines Hunde-Dopaminrezeptor-D4-Gens bindet.

14. Kit nach Anspruch 13, **dadurch gekennzeichnet, dass** das PCR-Primerpaar aus den Nukleotidsequenzen besteht, die durch die SEQ ID NO: 12 und die SEQ ID NO: 13 wiedergegeben sind.

## Revendications

1. Procédé de sélection de chiens candidats utiles, **caractérisé par** :
une étape d'étude du génotype de l'allèle dans l'exon 1 d'un gène du récepteur D4 de la dopamine de chien, et
une étape de sélection d'un chien avec des aptitudes génétiques bénéfiques pour les personnes en réalisant la sélection d'après ce génotype, où les chiens avec des aptitudes génétiques bénéfiques pour les personnes sont des chiens de service, et le génotype est un homozygote ou un hétérozygote d'un polynucléotide composé d'une séquence nucléotidique représentée par soit SEQ ID NO : 5 soit une séquence pratiquement identique à SEQ ID NO : 5.

2. Procédé de sélection de chiens candidats utiles selon la revendication 1, **caractérisé en ce que** les chiens de service sont des chiens guides d'aveugles, des chiens de sauvetage pour les catastrophes, des chiens d'aide, des chiens pour la détection de narcotiques, des chiens policiers, des chiens pour les malentendants, des chiens de troupeaux ou des chiens de chasse.

3. Procédé de sélection de chiens candidats utiles, **caractérisé par** :
une étape d'étude du génotype de l'allèle dans l'exon 1 d'un gène du récepteur D4 de la dopamine de chien, et
une étape de sélection d'un chien avec des aptitudes génétiques bénéfiques pour les personnes en réalisant la sélection d'après ce génotype, où les chiens avec des aptitudes génétiques bénéfiques pour les personnes sont des chiens de garde, et le génotype est un homozygote ou un hétérozygote d'un polynucléotide composé d'une séquence nucléotidique représentée par soit SEQ ID NO : 3 soit une séquence pratiquement identique à SEQ ID NO : 3.

4. Procédé de sélection de chiens candidats utiles selon l'une quelconque des revendications 1 à 3, **caractérisé en ce que** l'étape d'étude du génotype comprend une étape d'amplification de l'allèle en utilisant une paire d'amorces de PCR qui se lie à proximité des deux extrémités de l'allèle, et une étape de détermination du génotype de l'allèle basée sur les longueurs des produits de PCR obtenus par cette étape.

5. Procédé de sélection de chiens candidats utiles selon la revendication 4, **caractérisé en ce que** la paire d'amorces de PCR est composée des séquences nucléotidiques représentées par SEQ ID NO : 10 et SEQ ID NO : 11.

6. Kit de sélection de chiens candidats utiles, **caractérisé par** une paire d'amorces de PCR qui se lie à proximité des deux extrémités de l'allèle dans l'exon 1 d'un gène du récepteur D4 de la dopamine de chien.

7. Kit selon la revendication 6, **caractérisé en ce que** la paire d'amorces de PCR est composée des séquences nucléotidiques représentées par SEQ ID NO : 10 et SEQ ID NO : 11.

8. Procédé de sélection de chiens candidats utiles, **caractérisé par** :
une étape d'étude du génotype de l'allèle dans l'intron 2 d'un gène du récepteur D4 de la dopamine de chien, et
une étape de sélection d'un chien avec des aptitudes génétiques bénéfiques pour les personnes en réalisant la sélection d'après ce génotype.

9. Procédé de sélection de chiens candidats utiles selon la revendication 8, **caractérisé en ce que** l'étape d'étude du génotype comprend une étape d'amplification de l'allèle en utilisant une paire d'amorces de PCR qui se lie à proximité des deux extrémités de l'allèle, et une étape de détermination du génotype de l'allèle basée sur les longueurs des produits de PCR obtenus par cette étape.

10. Procédé de sélection de chiens candidats utiles selon la revendication 9, **caractérisé en ce que** la paire d'amorces de PCR est composée des séquences nucléotidiques représentées par SEQ ID NO : 12 et SEQ ID NO : 13.

11. Procédé de sélection de chiens candidats utiles selon l'une quelconque des revendications 8 à 10, **caractérisé en ce que** les chiens avec des aptitudes génétiques bénéfiques pour les personnes sont des chiens de service, et le génotype est un homozygote ou un hétérozygote d'un polynucléotide composé d'une séquence nucléotidique représentée par soit SEQ ID NO : 9 soit une séquence pratiquement identique à SEQ ID NO : 9.

12. Procédé de sélection de chiens candidats utiles selon l'une quelconque des revendications 8 à 10, **caractérisé en ce que** les chiens avec des aptitudes génétiques bénéfiques pour les personnes sont des chiens de garde ou des chiens de maison.

13. Kit de sélection de chiens candidats utiles, **caractérisé par** une paire d'amorces de PCR qui se lie à proximité des deux extrémités de l'allèle dans l'intron 2 d'un gène du récepteur D4 de la dopamine de chien.

14. Kit selon la revendication 13, **caractérisé en ce que** la paire d'amorces de PCR est composée des séquences nucléotidiques représentées par SEQ ID NO : 12 et SEQ ID NO : 13.


### SEQUENCE LISTING

<110> Gifu University
<120> Polynucleotides, polypeptides and method for screening for useful dog candidates.
<130> P2P2002381
<140>
   <141>
<150> JP2002-373006
   <151> 2002-12-24
<160> 13
<170> Patentln Ver. 2.1
<210> 1
   <211> 24
   <212> DNA
   <213> Canis familiaris
<220>
   <221> CDS
   <222> (1).. (24)
   <223> Polymorphic region of Dopamine Receptor D4 Exon1
<400> 1
   ggg acc ccg ggg acc ccg ggc gcg                                   24
   Gly Thr Pro Gly Thr Pro Gly Ala
     1               5
<210> 2
   <211> 8
   <212> PRT
   <213> Canis familiaris
<400> 2
   Gly Thr Pro Gly Thr Pro Gly Ala
     1               5
<210> 3
   <211> 267
   <212> DNA
   <213> Canis familiaris
<220>
   <221> CDS
   <222> (1)..(267)
   <223> Dopamine Receptor D4 Exon1 (Long form)
<400> 3
   atg ggg aac cgc agc gcc ggg gac gcc gac ggg ctg ctg gcg ggg cgc    48
   Met Gly Asn Arg Ser Ala Gly Asp Ala Asp Gly Leu Leu Ala Gly Arg
     1               5                  10                  15
   ggg ccg ggc gcg ggg acc ccg ggg acc ccg ggc gcg gcg gcg gcg ctg    96
   Gly Pro Gly Ala Gly Thr Pro Gly Thr Pro Gly Ala Ala Ala Ala Leu
                20                  25                  30
   gcg ggg ggc gtg ctg ctc atc ggc gcc gtg ctc gcg ggg aac gcg ctg    144
   Ala Gly Gly Val Leu Leu Ile Gly Ala Val Leu Ala Gly Asn Ala Leu
            35                  40                  45
   gtg tgc gcg agc gtg gcg gcc gag cgc gcc ctg cag acg ccc acc aac    192
   Val Cys Ala Ser Val Ala Ala Glu Arg Ala Leu Gln Thr Pro Thr Asn
        50                  55                  60
   tac ttc atc gtg agc ctg gcg gcc gcc gac cta ctc ctc gcc ctg ctc    240
   Tyr Phe Ile Val Ser Leu Ala Ala Ala Asp Leu Leu Leu Ala Leu Leu
    65                  70                  75                  80
   gtg ctg ccc ctg ttc gtc tac tcc gag                                267
   Val Leu Pro Leu Phe Val Tyr Ser Glu
                    85
<210> 4
   <211> 89
   <212> PRT
   <213> Canis familiaris
<400> 4
   Met Gly Asn Arg Ser Ala Gly Asp Ala Asp Gly Leu Leu Ala Gly Arg
     1               5                  10                  15
   Gly Pro Gly Ala Gly Thr Pro Gly Thr Pro Gly Ala Ala Ala Ala Leu
                20                  25                  30
   Ala Gly Gly Val Leu Leu Ile Gly Ala Val Leu Ala Gly Asn Ala Leu
            35                  40                  45
   Val Cys Ala Ser Val Ala Ala Glu Arg Ala Leu Gln Thr Pro Thr Asn
   50 55 60
   Tyr Phe Ile Val Ser Leu Ala Ala Ala Asp Leu Leu Leu Ala Leu Leu
   65 70 75 80
   Val Leu Pro Leu Phe Val Tyr Ser Glu
   85
<210> 5
   <211> 243
   <212> DNA
   <213> Canis familiaris
<220>
   <221> CDS
   <222> (1).. (243)
   <223> Dopamine Receptor D4 Exon1 (Short form)
<400> 5
   atg ggg aac cgc agc gcc ggg gac gcc gac ggg ctg ctg gcg ggg cgc 48
   Met Gly Asn Arg Ser Ala Gly Asp Ala Asp Gly Leu Leu Ala Gly Arg
   1 5 10 15
   ggg ccg ggc gcg gcg gcg gcg ctg gcg ggg ggc gtg ctg ctc atc ggc 96
   Gly Pro Gly Ala Ala Ala Ala Leu Ala Gly Gly Val Leu Leu Ile Gly
   20 25 30
   gcc gtg ctc gcg ggg aac gcg ctg gtg tgc gcg agc gtg gcg gcc gag 144
   Ala Val Leu Ala Gly Asn Ala Leu Val Cys Ala Ser Val Ala Ala Glu
   35 40 45
   cgc gcc ctg cag acg ccc acc aac tac ttc atc gtg agc ctg gcg gcc 192
   Arg Ala Leu Gln Thr Pro Thr Asn Tyr Phe Ile Val Ser Leu Ala Ala
   50 55 60
   gcc gac cta ctc ctc gcc ctg ctc gtg ctg ccc ctg ttc gtc tac tcc 240
   Ala Asp Leu Leu Leu Ala Leu Leu Val Leu Pro Leu Phe Val Tyr Ser
   65 70 75 80
   gag 243
   Glu
<210> 6
   <211> 81
   <212> PRT
   <213> Canis familiaris
<400> 6
   Met Gly Asn Arg Ser Ala Gly Asp Ala Asp Gly Leu Leu Ala Gly Arg
   1               5                   10                  15
   Gly Pro Gly Ala Ala Ala Ala Leu Ala Gly Gly Val Leu Leu Ile Gly
               20                  25                  30
   Ala Val Leu Ala Gly Asn Ala Leu Val Cys Ala Ser Val Ala Ala Glu
           35                  40                  45
   Arg Ala Leu Gln Thr Pro Thr Asn Tyr Phe Ile Val Ser Leu Ala Ala
       50                  55                  60
   Ala Asp Leu Leu Leu Ala Leu Leu Val Leu Pro Leu Phe Val Tyr Ser
   65                  70                  75                  80
   Glu
<210> 7
   <211> 17
   <212> DNA
   <213> Canis familiaris
<220>
   <221> intron
   <222> (1).. (17)
   <223> Polymorphic region of Dopamine Receptor D4 Intron 2
<400> 7
   cccgcccctc gccaggc 17
<210> 8
   <211> 133
   <212> DNA
   <213> Canis familiaris
<220>
   <221> intron
   <222> (1).. (133)
   <223> Dopamine Receptor D4 Intron 2 (Long form)
<400> 8
   gtgagccgcc ccgcgtcggc cccgcccccc gccaggcccc gcctcgcgac ccgcccctcg 60
   ccaggccccg ccctcgcccc gcccctcgcc aggccccgcc ccgcggcccg ccgccctcac 120
   tgcgcccccg cag 133
<210> 9
   <211> 116
   <212> DNA
   <213> Canis familiaris
<220>
   <221> intron
   <222> (1).. (116)
   <223> Dopamine Receptor D4 Intron 2 (Short form)
<400> 9
   gtgagccgcc ccgcgtcggc cccgcccccc gccaggcccc gcctcgcgac ccgccctcgc 60
   cccgcccctc gccaggcccc gccccgcggc ccgccgccct cactgcgccc ccgcag 116
<210> 10
   <211> 18
   <212> DNA
   <213> Artificial Sequence
<220>
   <223> Description of Artificial Sequence:PCR primer
<400> 10
   cgccatgggg aaccgcag 18
<210> 11
   <211> 19
   <212> DNA
   <213> Artificial Sequence
<220>
   <223> Description of Artificial Sequence:PCR primer
<400> 11
   cggctcacct cggagtaga 19
<210> 12
   <211> 19
   <212> DNA
   <213> Artificial Sequence
<220>
   <223> Description of Artificial Sequence:PCR primer
<400> 12
   gccatcagcg tggacaggt 19
<210> 13
   <211> 22
   <212> DNA
   <213> Artificial Sequence
<220>
   <223> Description of Artificial Sequence:PCR primer
<400> 13
   cgtcgttgag gccgcacagt ac 22